# EUROPÄISCHE PATENTSCHRIFT

(11) **EP 2 702 926 B1**
(45) Veröffentlichungstag und Bekanntmachung des Hinweises auf die Patenterteilung: **05.10.2016**
(21) Anmeldenummer: 13180854.5
(22) Anmeldetag: 19.08.2013
(51) Int. Cl.: A61B 1/005, F16H 25/20, A61B 1/00, A61B 17/00

(54) **Schaft für ein flexibles Endoskop oder ein flexibles endoskopisches Instrument**
Shaft for a flexible endoscope or a flexible endoscopic instrument
Tige pour un endoscope flexible ou un instrument endoscopique flexible

(30) Priorität: 31.08.2012 DE 102012108076
(43) Veröffentlichungstag der Anmeldung: 05.03.2014
(73) Patentinhaber: Karl Storz GmbH & Co. KG, 78532 Tuttlingen (DE); Storz Endoskop Produktions GmbH, 78532 Tuttlingen (DE)
(72) Erfinder: Kindler, Gereon, 78532 Tuttlingen (DE)

(56) Entgegenhaltungen:
- EP-A1- 1 902 665
- EP-A1- 2 189 103
- JP-A- H0 482 529
- US-A- 4 982 725
- US-A1- 2004 097 791
- US-A1- 2011 295 242

## Beschreibung

Die vorliegende Erfindung betrifft einen Schaft für ein flexibles Endoskop oder für ein flexibles endoskopisches Instrument nach dem Oberbegriff von Anspruch 1 sowie ein flexibles Endoskop und ein flexibles endoskopisches Instrument mit einem derartigen Schaft. Flexible Endoskope werden für eine Vielzahl von Anwendungen in Medizin und Technik eingesetzt. Derartige flexible Endoskope umfassen einen flexiblen lang erstreckten Schaft, der zur Einführung in einen Hohlraum, etwa einen körperinneren Hohlraum oder einen Hohlraum eines technischen Objekts, geeignet ist. In der Regel ist an der Spitze des Endoskopschafts ein Endoskopobjektiv zur Erzeugung eines Bildes einer Szene in dem beobachteten Hohlraum angeordnet. Zur Aufnahme und Weiterleitung des endoskopischen Bildes vom distalen (d.h. beobachtelfernen) zum proximalen (d.h. beobachternahen) Endbereich des Endoskops kann beispielsweise ein innerhalb des Schafts verlaufendes geordnetes Bündel von Lichtleitfasern vorgesehen sein oder auch ein elektronischer Bildaufnehmer, etwa ein CCD-Chip, der im Bereich des distalen Endes des Schafts angeordnet ist, und dessen Signale über innerhalb des Schafts verlaufende elektrische Leitungen zum proximalen Endbereich übertragen werden. Da in der Regel in dem beobachteten Hohlraum nicht ausreichend Licht vorhanden ist, kann ferner innerhalb des Schafts ein Lichtleitsystem angeordnet sein, um Licht an das distale Ende des Endoskops zu transportieren, wo es zur Beleuchtung des Hohlraums genutzt wird. Weiterhin kann der Endoskopschaft einen oder mehrere Arbeitskanäle zur Durchführung von endoskopischen Arbeitsinstrumenten vom proximalen zum distalen Endbereich des Schafts enthalten, mit deren Hilfe innerhalb des Hohlraums Manipulationen ausgeführt werden können.

Es sind auch endoskopische Instrumente bekannt, die einen flexiblen lang erstreckten Schaft umfassen, der ebenfalls zur Einführung in einen Hohlraum, etwa einen körperinneren Hohlraum oder einen Hohlraum eines technischen Objekts, geeignet ist. Ein derartiges flexibles endoskopisches Instrument kann zur Durchführung von Manipulationen in dem Hohlraum verwendbar sein und hierfür beispielsweise als Fassinstrument zum Greifen und Manipulieren von Gewebe oder Gegenständen in dem körperinneren Hohlraum bzw. in dem Hohlraum eines technischen Objekts ausgebildet sein. Hierfür ist am distalen Ende des flexiblen Schafts ein Werkzeug angeordnet, das über ein innerhalb des Schafts verlaufendes Übertragungsmittel vom proximalen Ende des Schafts her bedient werden kann. Ein solches flexibles endoskopisches Instrument weist in der Regel keine eigene Optik zur Aufnahme eines endoskopischen Bildes auf, kann aber insbesondere gemeinsam mit einem flexiblen Endoskop verwendbar sein.

Häufig ist es wünschenswert, das distale Ende des Schafts, d.h. die Spitze des Endoskops bzw. des endoskopischen Instruments, abwinkeln zu können, um die Einführung des Endoskops bzw. des endoskopischen Instruments durch einen nicht geradlinig verlaufenden Kanal zu erleichtern, um die Spitze innerhalb eines Hohlraums in einer seitlichen Richtung bewegen zu können und um die Blickrichtung einer in der Endoskopspitze angeordneten Optik bzw. die Arbeitsrichtung eines an der Spitze des endoskopischen Instruments angeordneten Werkzeugs verändern zu können. Hierfür weist der Schaft einen steuerbaren Abschnitt, insbesondere einen steuerbaren Endabschnitt, auf, der aktiv um einen gewünschten Betrag in eine gewünschte Richtung abgewinkelt werden und hierfür vom proximalen Ende des Endoskops bzw. des endoskopischen Instruments her gesteuert werden kann. Der Schaft für ein flexibles endoskopisches Instrument muss nicht selbst ein Werkzeug und ein Übertragungsmittel aufweisen, sondern kann beispielsweise einen Arbeitskanal umfassen, in den ein flexibles endoskopisches Arbeitsinstrument, das nicht aktiv abwinkelbar ist und das ein solches Werkzeug aufweist, bis zum distalen Ende des Schafts und ggf. darüber hinaus eingeführt werden kann, so dass das flexible Arbeitsinstrument mit Hilfe des Schafts abgewinkelt werden kann.

Um eine steuerbare Abwinkelung eines Abschnitts eines Schafts eines flexiblen Endoskops zu ermöglichen, ist es bekannt, diesen mit einer Grundstruktur aus einzelnen gegeneinander schwenkbaren Segmenten auszubilden, die durch im Endoskopschaft geführte Seilzüge bzw. Bowdenzüge betätigt werden können. Zur Betätigung sind insbesondere am Endoskophandgriff angeordnete Handräder vorgesehen. Gemäß US 2005/0131279 A1 ist jedes Segment eines steuerbaren Abschnitts des Endoskopschafts relativ zum nächsten bzw. vorhergehenden Segment schwenkbar gelagert. Zum Abwinkeln des betreffenden Schaftabschnitts bzw. der Endoskopspitze sind vier Seilzüge vorgesehen, die von einer proximal angeordneten Steuerungseinrichtung bis zur Endoskopspitze geführt sind. Die Seilzüge verlaufen im Randbereich der Segmente und sind bezüglich einer Längsachse jeweils um 90° zueinander versetzt, so dass eine Steuerung der Endoskopspitze in eine gewünschte Richtung durch Aufrollen der entsprechenden Seile in der Steuerungseinrichtung bewirkt werden kann.

Die Seile derartiger Seilzüge neigen jedoch dazu, sich nach mehrfachem Gebrauch zu längen, was die Steuerbarkeit der Endoskopspitze beeinträchtigt oder eine regelmäßige Nachspannung der Seilzüge erforderlich macht. Nachstellvorrichtungen zur Einstellung der Spannung der Seilzüge sind beispielsweise aus DE 29 14 748 C2 und US 4,203,430 bekannt; derartige Nachstellvorrichtungen sind jedoch mit einem erhöhten konstruktiven und Bedienungsaufwand verbunden. Die erforderliche dauerhafte Spannung der Seilzüge kann ferner zu einer Verkürzung des Schafts führen, was die Steuerbarkeit der Endoskopspitze sowie überhaupt die Eigenschaften des Endoskopschafts, etwa die Flexibilität oder die Obelflächenbeschaffenheit, nachteilig beeinflussen kann. Die erzielbare Abwinkelung hängt dabei außerdem von der Krümmung des gesamten Schaftabschnitts ab, durch den die Seilzüge geführt sind, so dass kein eindeutiger Zusammenhang zwischen der Stellung eines Handrads, mit dem ein Seilzug aufgerollt werden kann, und dem erzielten Ablenkwinkel der Endoskopspitze besteht. Krümmungen des Endoskopschafts verursachen ferner eine erhöhte Reibung der Seilzüge, was die Bedienbarkeit der Abwinkelung ebenfalls erschwert. Schließlich können die Seilzüge, insbesondere bei längeren Schäften, aufgrund der Reibung und eines entsprechenden Verschleißes reißen.

In JP 07134253 A ist ein Endoskop mit einem krümmbaren Abschnitt offenbart, der eine Mehrzahl von gelenkig miteinander verbundenen Segmenten umfasst, die Gewindemuttern aufweisen, die mit Bolzen zusammenwirken, die mit einer flexiblen Welle verbunden sind. Nahe einem proximalen Ende des krümmbaren Abschnitts ist im Schaft des Endoskops ein Mikromotor angeordnet, der die flexible Welle zur Steuerung einer Abwinkelung des krümmbaren Abschnitts in Rotation versetzt. Hiermit ist ein relativ hoher konstruktiver Aufwand verbunden. Ferner ist die Belastbarkeit der Welle mit den damit verbundenen Gewindemuttern nicht immer ausreichend. Außerdem ist die Anordnung eines Mikromotors in einem Endoskopschaft mit geringem Durchmesser, innerhalb dessen weitere Leitungen bzw. Kanäle verlaufen, nicht ohne Weiteres möglich und aus auch Gründen der Zugänglichkeit des Motors zu Reparatur- und Wartungszwecken sowie ggf. zur Reinigung und Sterilisierung mit einem hohen Aufwand verbunden.

Die EP1902665A1 offenbart ein flexibles Endoskop mit einem proximalen Abschnitt, in dem ein Motor angeordnet ist, der in einem daran angeschlossenen flexiblen Schaft in dessen proximalem Endbereich eine kurze Torsionswelle und einen daran angeordneten Gewindetrieb antreibt. Der Gewindetrieb dient zur längsaxialen Verstellung von Zugseilen, die durch den ganzen flexiblen Schaft verlaufen und einen distalen abwinkelbaren Teil betätigen.

Aus der JPH04-82429A ist ein flexibles Endoskop bekannt, mit einem flexiblen Schaftabschnitt und einem distal davon angeordneten abwinkelbaren Schaftabschnitt, wobei sich zwischen diesen Abschnitten ein kurzer, im Durchmesser erweiterter Abschnitt erstreckt. In diesem Abschnitt sind Motoren und kurze Torsionswellen zur Betätigung eines Gewindtriebs angeordnet. Durch die Bewegung des Gewindetriebs werden Zugseile im distalen Schaftabschnitt axial bewegt und der Schaftabschnitt abgewinkelt.

Es ist die Aufgabe der vorliegenden Erfindung, einen Schaft für ein flexibles Endoskop oder ein flexibles endoskopisches Instrument sowie ein flexibles Endoskop und ein flexibles endoskopisches Instrument anzugeben, wobei die oben genannten Nachteile vermieden werden und wobei der Schaft insbesondere genau steuerbar, einfach aufgebaut, dauerhaft und robust ist.

Diese Aufgabe wird durch einen Schaft gemäß Anspruch 1 sowie durch ein flexibles Endoskop gemäß Anspruch 9 und ein flexibles endoskopisches Instrument gemäß Anspruch 10 gelöst. Vorteilhafte Weiterbildungen der Erfindung ergeben sich aus den Unteransprüchen.

Ein erfindungsgemäßer Schaft für ein flexibles Endoskop oder für ein flexibles endoskopisches Instrument ist lang erstreckt und zur Einführung in einen Hohlraum, beispielsweise einen körperinneren Hohlraum oder einen Hohlraum eines technischen Objekts, ausgebildet. Das flexible Endoskop kann insbesondere einen an einem proximalen Ende des Schafts angeordneten Handgriff umfassen, der Bedienelemente und/oder Anschlüsse für weitere Geräte, etwa eine Saug- und Spüleinrichtung, eine Lichtquelle oder eine Kameraeinheit, aufweisen kann. Der Handgriff kann auch eine Kamera enthalten. Das flexible endoskopische Instrument kann insbesondere einen an einem proximalen Ende des Schafts angeordneten Handgriff mit einem Betätigungselement für ein am distalen Ende des Schafts angeordnetes Werkzeug aufweisen. Das flexible Endoskop bzw. das flexible endoskopische Instrument ist insbesondere zur Verwendung bei medizinischen, insbesondere chirurgischen Eingriffen oder für technische Anwendungen ausgebildet.

Der Schaft umfasst einen ersten Schaftabschnitt sowie einen distalseitig des ersten Schaftabschnitts angeordneten zweiten Schaftabschnitt, der insbesondere an einen distalen Endbereich des ersten Schaftabschnitts anschließen kann. Der Schaft kann weitere Schaftabschnitte umfassen. Der zweite Schaftabschnitt kann beispielsweise als abwinkelbare Endoskopspitze ausgebildet sein. Der zweite Schaftabschnitt ist relativ zu dem distalen Endbereich des ersten Schaftabschnitts abwinkelbar. Insbesondere ist der zweite Schaftabschnitt aktiv krümmbar ausgebildet, während der erste Schaftabschnitt zumindest teilweise flexibel ausgebildet, jedoch nicht aktiv krümmbar ist.

Innerhalb des zweiten Schaftabschnitts ist mindestens ein in Längsrichtung des zweiten Schaftabschnitts wirkendes Zugmittel angeordnet. Der Begriff "Zugmittel" umfasst hier beispielsweise einen Draht oder ein Zugseil, das eine Zugkraft ausübt, kann aber auch als Schubmittel ausgebildet sein, beispielsweise als flexible Stange oder als steifer Draht oder auch als ein in einer Hülle geführter Draht, etwa als Bowdenzug, der zur Übertragung sowohl von Zug- als auch Schubkräften in Längsrichtung des zweiten Schaftabschnitts geeignet ist. Der zweite Schaftabschnitt ist relativ zu einem distalen Endbereich des ersten Schaftabschnitts durch eine Längsverstellung des Zugmittels abwinkelbar. Hierfür ist das Zugmittel innerhalb des zweiten Schaftabschnitts längsverschiebbar geführt und mit einem distalen Endbereich des zweiten Schaftabschnitts verbunden. Insbesondere wirkt das Zugmittel derart mit einer Stützstruktur des zweiten Schaftabschnitts zusammen, dass eine Längenänderung oder eine Längsverschiebung des Zugmittels eine Abwinkelung des zweiten Schaftabschnitts bewirkt, insbesondere eine Krümmung des zweiten Schaftabschnitts. Das Zugmittel kann insbesondere von einem proximalen bis zu einem distalen Endbereich des zweiten Schaftabschnitts geführt und nur im distalen Endbereich mit der Stützstruktur verbunden sein. Die Stützstruktur kann beispielsweise aus einer Abfolge miteinander gelenkig verbundener Segmente bestehen, wobei das Zugmittel gegenüber den Gelenken seitlich versetzt an mindestens einem Endsegment der Stützstruktur angreift.

Erfindungsgemäß ist mindestens ein Gewindetrieb zur Längsverstellung des mindestens einen Zugmittels angeordnet. Der Gewindetrieb kann hierfür etwa ein Teil des Zugmittels sein oder an diesem angreifen. Ein Gewindetrieb umfasst beispielsweise eine Gewindestange mit einem Außengewinde und eine Mutter mit einem entsprechenden Innengewinde, die derart zusammenwirken, dass durch eine Drehung der Mutter relativ zur Gewindestange die Gewindestange relativ zur Mutter in Längsrichtung der Gewindestange verschoben wird, um eine Längsverstellung des Zugmittels zu bewirken.

Der zur Längsverstellung des mindestens einen Zugmittels angeordnete mindestens eine Gewindetrieb ist über mindestens eine innerhalb des ersten Schaftabschnitts verlaufende Torsionswelle antreibbar. Durch die im ersten Schaftabschnitt angeordnete Torsionswelle kann eine von proximalseitig des ersten Schaftabschnitts angeordneten Betätigungsmitteln erzeugte Betätigungsbewegung zu dem mindestens einen Gewindetrieb, der etwa im zweiten Schaftabschnitt oder in einem Endbereich des ersten Schaftabschnitts angeordnet sein kann, übertragen werden, um den Gewindetrieb anzutreiben und dadurch eine Längsverstellung des mindestens einen Zugmittels zu bewirken. Die Betätigungsmittel können beispielsweise als ein an einem Handgriff angeordnetes Handrad oder als in einem Handgriff angeordneter Motor ausgebildet sein. Durch die Betätigungsmittel kann eine Rotationsbewegung der Torsionswelle erzeugt werden. Die Torsionswelle ist erfindungsgemäß lang erstreckt, durchgehend vom proximalen zum distalen Endbereich des ersten Schaftabschnitts und zumindest abschnittsweise flexibel ausgebildet. Die Torsionswelle kann innerhalb des ersten Schaftabschnitts in einem Randbereich des Querschnitts geführt sein, so dass eine ausreichende Querschnittsfläche für die Durchführung weiterer Kanäle bzw. elektrischer und Lichtleitungen verbleibt. Durch die Rotation der mit dem mindestens einen Gewindetrieb gekoppelten mindestens einen Torsionswelle ist somit die Längsverstellung des Zugmittels zur Steuerung der Abwinkelung des zweiten Schaftabschnitts steuerbar.

Dadurch, dass ein Gewindetrieb zur Längsverstellung des Zugmittels vorgesehen ist, kann eine genau steuerbare Abwinkelung des zweiten Schaftabschnitts des Schafts mit ausreichender Kraft realisiert werden. Ferner ist ein Gewindetrieb einfach aufgebaut, robust und kostengünstig herstellbar. Weiterhin kann dadurch, dass der Gewindetrieb über mindestens eine innerhalb des ersten Schaftabschnitts verlaufende Torsionswelle antreibbar ist, eine Betätigungsbewegung zur Steuerung der Abwinkelung des zweiten Schaftabschnitts auf einfache Weise durch einen mit einem geringen Durchmesser ausgebildeten Schaft übertragen werden. Insbesondere wird die Betätigungsbewegung direkter zum zweiten Schaftabschnitt übertragen, als dies mit Seil- bzw. Bowdenzügen möglich wäre. Dabei wird keine Zug- oder Druckkraft auf den ersten Schaftabschnitt ausgeübt, der in der Regel den überwiegenden Teil der Gesamtlänge des Schafts ausmacht, so dass eine Stauchung des Schafts vermieden wird. Ferner ist keine Nachspannung von Seilzügen, die sich im Laufe der Zeit verlängern könnten, erforderlich. Weiterhin ist die bis zum zweiten Schaftabschnitt übertragene Betätigungsbewegung weitgehend unabhängig von der Länge und der Krümmung des ersten Schaftabschnitts, so dass die Steuerbarkeit der Abwinkelung des zweiten Schaftabschnitts verbessert ist. Auf diese Weise wird insbesondere ein einfach aufgebauter flexibler Schaft für ein flexibles Endoskop bzw. für ein flexibles endoskopisches Instrument geschaffen, bei dem ein abwinkelbarer distaler Schaftabschnitt unabhängig von einer Krümmung eines proximalen Schaftabschnitts mit proximal angeordneten Betätigungsmitteln steuerbar ist.

Gemäß einer bevorzugten Ausführungsform der Erfindung ist das mindestens eine Zugmittel als Seilzug ausgebildet, der zur Steuerung seiner Längsverstellung mit einem distalen Ende der Torsionswelle über den Gewindetrieb verbunden ist. Der Seilzug ist innerhalb des zweiten Schaftabschnitts längsverschiebbar geführt und an seinem distalen Ende mit einem distalen Endbereich des zweiten Schaftabschnitts verbunden. Das proximale Ende des Seilzugs ist mit dem Gewindetrieb verbunden. Der Gewindetrieb kann in einem proximalen Endbereich des zweiten Schaftabschnitts, in einem distalen Endbereich des ersten Schaftabschnitts, übergreifend in beiden Schaftabschnitten oder in einem Übergangsabschnitt zwischen dem ersten und dem zweiten Schaftabschnitt angeordnet sein. Der Übergangsabschnitt muss nicht unbedingt flexibel ausgebildet sein. Dadurch, dass eine Abwinklung des zweiten Schaftabschnitts durch eine Längsverstellung eines Seilzugs erfolgt, dessen proximales Ende durch den Gewindetrieb in Längsrichtung verschiebbar ist, kann auf besonders einfache und wirksame Weise die Abwinkelung des zweiten Schaftabschnitts bewirkt werden.

Vorzugsweise umfasst der Gewindetrieb eine Gewindestange, die mit einer Gewindemutter, die mit dem distalen Ende der Torsionswelle drehfest verbunden ist, zur Längsverstellung des Seilzugs zusammenwirkt. Die Mutter ist auf die Gewindestange aufgesetzt und weist ein mit dem Außengewinde der Gewindestange zusammenwirkendes Innengewinde auf. Durch eine Verdrehung der Mutter relativ zur Gewindestange können die Mutter und die Gewindestange in Längsrichtung relativ zueinander verschoben werden. Insbesondere ist der Gewindetrieb im distalen Endbereich des ersten Schaftabschnitts angeordnet. Der distale Endbereich des ersten Schaftabschnitts kann starr oder flexibel ausgebildet sein. Die Gewindestange kann flexibel sein, ist jedoch vorzugsweise starr ausgebildet. Ein solcher, insbesondere nur eine Gewindestange und nur eine einzige mit dieser zusammenwirkende Mutter umfassender Gewindetrieb ist einfach aufgebaut und robust und ermöglicht eine genaue Steuerung der Abwinkelung des zweiten Schaftabschnitts.

In besonders vorteilhafter Weise ist die Gewindestange im distalen Endbereich des ersten Schaftabschnitts längsverschiebbar, jedoch drehfest geführt und mit ihrem distalen Ende mit dem proximalen Ende des innerhalb des zweiten Schaftabschnitts geführten Seilzugs verbunden. Die Mutter ist drehfest mit dem distalen Ende der Torsionswelle verbunden und im distalen Endbereich des ersten Schaftabschnitts drehbar gelagert und in Längsrichtung abgestützt. Eine Rotation der Torsionswelle in einer ersten Richtung führt zu einer Bewegung des proximalen Endes des Seilzugs in proximaler Richtung und zu einer entsprechenden Abwinkelung des zweiten Schaftabschnitts, während eine Rotation der Torsionswelle in entgegengesetzter Richtung eine Bewegung des proximalen Endes des Seilzugs in distaler Richtung bewirkt und damit eine Verringerung der Abwinkelung bzw. eine entgegengesetzte Abwinkelung ermöglicht. Hierdurch ist auf besonders einfache Weise eine Umsetzung der Rotationsbewegung der Torsionswelle in eine Längsverstellung des Seilzugs zur Steuerung der Abwinkelung des zweiten Schaftabschnitts möglich.

Gemäß einer weiteren bevorzugten Ausführungsform der Erfindung ist das Zugmittel zumindest abschnittsweise selbst als Gewindestange des mindestens einen Gewindetriebs ausgebildet, die zumindest abschnittsweise flexibel ist. Die Gewindestange ist somit innerhalb des zweiten Schaftabschnitts längsverschiebbar geführt und mit einem distalen Endbereich des zweiten Schaftabschnitts verbunden, vorzugsweise nur mit einem distalen Endbereich des zweiten Schaftabschnitts.

Insbesondere ist die Gewindestange drehfest im Schaft gelagert und wirkt mit einer Gewindemutter zusammen, die mit dem distalen Ende der Torsionswelle drehfest verbunden ist. Auf einem proximalen Endbereich der Gewindestange ist die Gewindemutter aufgesetzt, die mit der Torsionswelle drehbar gelagert und durch diese antreibbar ist, jedoch gegen eine Längsverschiebung relativ zum Endoskopschaft abgestützt ist. Durch eine Rotation der Torsionswelle rotiert daher die Mutter relativ zur Gewindestange, wodurch der proximale Endbereich der Gewindestange in Längsrichtung verschoben wird. Dadurch wird die wirksame Länge des im zweiten Schaftabschnitt geführten Zugmittels, das zumindest abschnittsweise durch die Gewindestange gebildet wird, verändert und damit eine Längsverstellung des Zugmittels bewirkt. Ein derartiger Gewindetrieb umfasst nur eine Gewindestange und nur eine einzige mit dieser zusammenwirkende Mutter und ist daher besonders einfach aufgebaut, wobei ferner ein Seilzug zur Abwinkelung des zweiten Schaftabschnitts entfallen kann. Hierdurch wird auf eine besonders einfache Weise eine Steuerung der Abwinkelung des zweiten Schaftabschnitts durch eine Rotation der Torsionswelle ermöglicht.

Vorzugsweise weist der zweite Schaftabschnitt eine Mehrzahl von Zugmitteln auf, die einander paarweise gegenüber liegend angeordnet sind. Bei einer Abwinkelung des zweiten Schaftabschnitts wirken somit jeweils zwei Zugmittel einander entgegen, wobei die jeweiligen Längsverstellungen der Zugmittel einander entgegengerichtet sind. Hierdurch kann eine Abwinkelung in unterschiedliche Richtungen mit einer besonders hohen Genauigkeit und einer stets ausreichenden Kraft erzielt werden, da in jeder Abwinkelungsrichtung eines der Zugmittel verkürzt bzw. das distale Ende eines der Zugmittel in proximale Richtung gezogen wird.

Insbesondere können zwei in Bezug auf eine Längsachse des zweiten Schaftabschnitts um 90° zueinander versetzte Paare von Zugmitteln vorgesehen sein, um eine Abwinkelung des zweiten Schaftabschnitts in aufeinander senkrecht stehenden Richtungen zu ermöglichen. Hierdurch ist auf eine besonders einfache Weise eine Abwinkelung, von einem Benutzer gesehen, sowohl nach links oder rechts als auch nach oben oder unten sowie durch eine gleichzeitige Betätigung der Paare von Zugmitteln eine Abwinkelung in jede dazwischen liegende Richtung möglich.

Alternativ können beispielsweise drei bezüglich der Längsachse um jeweils etwa 120° versetzte Zugmittel vorgesehen sein. Auch hierdurch ist eine Abwinkelung in alle Richtungen erreichbar.

In vorteilhafter Weise ist eine Mehrzahl von Torsionswellen vorgesehen, die mit der Mehrzahl von Zugmitteln zur Steuerung einer Abwinkelung des zweiten Schaftabschnitts zusammenwirken. Insbesondere kann jede Torsionswelle mit jeweils einem Zugmittel bzw. jeweils einem Gewindetrieb verbunden sein. Hierdurch wird auf einfache Weise eine Abwinkelung des zweiten Schaftabschnitts in unterschiedliche Richtungen mit einer besonders hohen Genauigkeit ermöglicht.

Gemäß einer bevorzugten Ausführungsform der Erfindung sind die mehreren Torsionswellen über ein Zahnradgetriebe derart miteinander verbunden, dass einander gegenüber liegende Zugmittel jeweils gegensinnig bewegbar sind. Insbesondere kann das Zahnradgetriebe an einem proximalen Ende der Torsionswelle angeordnet sein, wobei jeweils zwei einander gegenüber liegenden Gewindetrieben zugeordnete Torsionswellen miteinander gekoppelt sind. Durch das Zahnradgetriebe ist eine derartige Kopplung möglich, dass eine Verlängerung eines Zugmittels zu einer gleichzeitigen Verkürzung eines diesem gegenüber liegenden und diesem entgegenwirkenden Zugmittels führt bzw. eine Verschiebung eines Zugmittels in proximaler Richtung zu einer Verschiebung eines diesem gegenüber liegenden und diesem entgegenwirkenden Zugmittels in distaler Richtung führt. Hierfür kann beispielsweise jede der beiden einander entgegenwirkenden Torsionswellen an ihrem proximalen Ende drehfest ein Zahnrad tragen, wobei die beiden Zahnräder miteinander kämmen, so dass die Torsionswellen stets gegensinnig rotieren und die jeweiligen Gewindetriebe, die in diesem Fall gleichartig ausgestaltet sein können, gegenläufig angetrieben werden und gegenläufige Längsverstellungen der Zugmittel bewirken. Weisen die beiden entsprechenden Gewindetriebe unterschiedliche Gewinderichtungen auf, also einer ein Links- und einer ein Rechtgewinde, so kann eine ungerade Anzahl von Zahnrädern zwischen die beiden mit den Torsionswellen verbundenen Zahnräder zwischengeschaltet sein, so dass die beiden Torsionswellen gleichsinnig rotieren und über die unterschiedlichen Gewindetriebe gegenläufige Längenänderungen der Zugmittel bewirken. Hierdurch wird die Bedienbarkeit und Steuerbarkeit der Abwinkelung des zweiten Schaftabschnitts weiter verbessert.

In vorteilhafter Weise weist der mindestens eine Gewindetrieb, insbesondere eine Gewindestange des Gewindetriebs, eine konstante Gewindesteigung auf. Hierdurch wird ein linearer Zusammenhang zwischen dem Drehwinkel bzw. der Zahl der Umdrehungen der Torsionswelle einerseits und der dadurch bewirkten Längsverstellung des Zugmittels und damit der Abwinkelung des zweiten Schaftabschnitts andererseits ermöglicht. Ferner kann hierdurch ein spannungsarmes Entgegenwirken einander gegenüberliegender Gewindetriebe und der damit gekoppelten Torsionswellen zur Abwinkelung des zweiten Schaftabschnitts in unterschiedliche Richtungen erreicht werden. Eine geringe Gewindesteigung des Gewindetriebs ermöglicht dabei eine besonders feinfühlige Steuerung der Abwinkelung, während eine größere Gewindesteigung eine besonders rasche Verstellung der Abwinkelung mit relativ wenigen Umdrehungen der Torsionswelle ermöglicht.

Ein erfindungsgemäßes Endoskop umfasst einen oben beschriebenen Schaft, der als Endoskopschaft ausgebildet ist. Durch den Schaft können insbesondere Lichtleiter, Bildleiter, Saug- bzw. Spülkanäle und/oder einen oder mehrere Arbeitskanäle für endoskopische Arbeitsinstrumente verlaufen.

Ein erfindungsgemäßes endoskopisches Instrument umfasst einen langerstreckten Schaft, der wie oben beschrieben ausgebildet ist. Ferner kann ein am distalen Ende des Schafts des endoskopischen Instruments ein Werkzeug angeordnet sein, das über ein innerhalb des Schafts verlaufendes Übertragungsmittel, etwa ein Zugelement, von proximalen Ende des Schafts her, etwa mit Hilfe von proximal angeordneten Griffen, bedient werden kann. Der Schaft des endoskopischen Instruments kann beispielsweise Saug- bzw. Spülkanäle sowie einen Arbeitskanal umfassen, durch den ein nicht aktiv steuerbares endoskopisches Arbeitsinstrument einführbar ist.

In bevorzugter Weise ist das flexible Endoskop bzw. das flexible endoskopische Instrument derart ausgebildet, dass die mindestens eine Torsionswelle über mindestens ein in einem proximalen Bereich des Endoskops bzw. des endoskopischen Instruments, insbesondere an einem Handgriff, angeordnetes Bedienelement, das etwa als Handrad, Hebel oder Kurbel ausgebildet sein kann, und/oder einen Motor, der beispielsweise in dem Handgriff angeordnet sein kann, betätigt werden kann. Sowohl durch das Bedienelement als auch durch den Motor kann die Torsionswelle in einer für eine genaue Steuerung der Abwinkelung ausreichenden Weise in Rotation versetzt werden. Der Motor kann aber auch beispielsweise in einer vom Endoskop bzw. endoskopischen Instrument sowie ggf. vom Handgriff separaten Motoreinheit angeordnet sein, wobei die Rotationsbewegung über eine flexible Welle zum Endoskopschaft übertragbar sein kann. Die Betätigung über einen Motor, der von einer Steuerungseinrichtung angesteuert werden kann, ist insbesondere auch dann vorteilhaft, wenn die Zugmittel nicht paarweise einander gegenüberliegend, sondern beispielsweise um 120° gegeneinander versetzt angeordnet sind.

Gemäß einer weiteren bevorzugten Ausführungsform der Erfindung umfasst das flexible Endoskop bzw. das flexible endoskopische Instrument eine Handhabe, insbesondere einen Handgriff, wobei der Schaft über eine Kupplung mit der Handhabe lösbar verbindbar ist. Ein endoskopisches System kann somit eine Mehrzahl von Schäften unterschiedlicher Maße, insbesondere unterschiedlicher Durchmesser und Längen, sowie ggf. unterschiedlichen Aufbaus umfassen, die mit einer Handhabe, die beispielsweise den Antrieb für die Abwinkelung und eine Kamera umfassen kann, verbindbar sind. Dadurch, dass beim Ankoppeln des Schafts an die Handhabe keine in Längsrichtung des Schafts verlaufenden Seilzüge mit der Handhabe verbunden werden müssen, ist es nicht notwendig, diese nach dem Ankoppeln neu zu justieren bzw. zu spannen. Durch die Übertragung der Betätigungsbewegung zum Antrieb des Gewindetriebs über eine Torsionswelle wird demgegenüber eine einfachere Verbindung ermöglicht, die lediglich drehfest ausgestaltet sein muss und in Längsrichtung einfach trennbar sein kann. Der Antrieb und eine Steuerungseinrichtung sind in der Handhabe angeordnet. Hierdurch wird ein Endoskop bzw. ein endoskopisches Instrument und/oder ein endoskopisches System geschaffen, das besonders vielseitig einsetzbar ist, jedoch aufgrund der mit einer Mehrzahl von Schäften verwendbaren Handhabe relativ kostengünstig ist und beim Wechseln der Schäfte einen geringen Bedienungsaufwand erfordert.

In besonders bevorzugter Weise umfasst die Handhabe einen motorischen Antrieb der mindestens einen Torsionswelle bzw. des Gewindetriebs. Beim Abkoppeln des Schafts von der Handhabe wird eine rotatorische Position der mindestens einen Torsionswelle gespeichert, und eine Torsionswellenkupplung der Handhabe ist zum Ankoppeln des Schafts derart ansteuerbar, dass diese bei einem Ankoppeln eine der gespeicherten Rotationsstellung der Torsionswelle entsprechende rotatorische Position einnimmt. Hierdurch wird ein besonders einfaches Ankoppeln des Schafts an die Handhabe ermöglicht. Ferner ermöglicht die Speicherung der Rotationsstellung der Torsionswelle eine besonders genaue Steuerung der Abwinkelung des zweiten Schaftabschnitts. Aufgrund der Rotationsstellung der Torsionswelle kann eine jeweils aktuelle Rotationsstellung eines Gewindetriebs ermittelt und damit die Abwinkelung genau gesteuert werden. Hierdurch kann von der Steuerungseinrichtung auch die jeweils aktuelle Abwinkelungsstellung des zweiten Schaftabschnitts ermittelt werden, so dass etwa bei einer virtuellen Darstellung des Endoskops bzw. des endoskopischen Instruments und des beobachteten Hohlraums die räumliche Position der Endoskopspitze richtig dargestellt werden kann.

Die rotatorische Position der mindestens einen Torsionswelle, die diese beim Trennen von der entsprechenden Motorwelle einnimmt, ist in vorteilhafter Weise in einem dem Schaft zugeordneten Speicher speicherbar. Hierdurch kann erreicht werden, dass beim Verbinden des Schafts mit einer Handhabe die Kupplung der Motorwelle in die richtige Position gedreht werden kann und die Steuerungseinrichtung die notwendigen Informationen für eine genaue Steuerung der Abwinkelung hat, auch in dem Fall, dass der Schaft mit einer anderen Handhabe verbunden wird.

Zur Ermittlung der rotatorischen Position der Torsionswelle beim Trennen von der Handhabe kann ein Sensor, etwa ein Potentiometer oder ein anderer Drehgeber, insbesondere ein Absolutwertgeber, etwa ein optischer Multiturn-Drehgeber, vorgesehen sein, der insbesondere in einem proximalen Endbereich des Schafts angeordnet ist. Durch den Sensor werden die Umdrehungen und der zurückgelegte Winkel der Torsionswelle gemessen; der Sensor kann bei einer Trennung vom Antrieb in der entsprechenden Drehposition verharren, so dass ein Auslesen jederzeit möglich ist. Auf diese Weise können die beim Betrieb des Endoskops bzw. des endoskopischen Instruments zur Abwinkelung des zweiten Schaftabschnitts durchgeführten Umdrehungen gezählt und die rotatorische Position der Torsionswelle ermittelt werden.

Bei der Verwendung eines erfindungsgemäßen Endoskops bzw. eines erfindungsgemäßen endoskopischen Instruments wird somit zum Steuern eines abwinkelbaren Schaftabschnitts, insbesondere einer abwinkelbaren Spitze des Endoskops oder zum Steuern der Arbeitsrichtung eines an der Spitze des endoskopischen Instruments angeordneten Werkzeugs, mit Hilfe eines Bedienelements oder durch einen Motor insbesondere eine in einem ersten, proximalseitig angeordneten Schaftabschnitt verlaufende Torsionswelle gedreht und hierdurch ein in einem zweiten Abschnitt des Schafts verlaufendes Zugmittel in seiner Längsrichtung verstellt, d.h. verkürzt oder verlängert bzw. verschoben. Durch die Längsverstellung des Zugmittels wird der zweite Schaftabschnitt gekrümmt und auf diese Weise die Endoskopspitze bzw. die Spitze des endoskopischen Instruments abgewinkelt.

Soll ein anderer Endoskopschaft mit derselben Handhabe des flexiblen Endoskops oder derselbe Schaft mit einer anderen Handhabe verwendet werden, so wird der mit der Handhabe verbundene Schaft von dieser getrennt; da die Torsionswelle nur drehfest mit einer der Handhabe zugeordneten Motorwelle verbunden sein muss, ist dies auf besonders einfache Weise möglich. Entsprechendes gilt für den Schaft und die Handhabe eines flexiblen endoskopischen Instruments. Die Rotation der Torsionswelle kann durch einen Sensor ermittelbar sein und in einem im proximalen Endbereich des Schafts angeordneten Speicher beim Trennen des Schafts von der Handhabe gespeichert werden. Beim Verbinden eines anderen Schafts mit der Handhabe bzw. des Schafts mit einer anderen Handhabe wird die in dem Speicher des zu verbindenden Schafts gespeicherte rotatorische Position ausgelesen, etwa elektrisch, mechanisch oder auch über drahtlose Übertragungsmittel, und die Motorwelle in eine geeignete Position gebracht, so dass die Verbindung auf einfache Weise erfolgen kann. Ferner ermöglicht das Auslesen der rotatorischen Position eine Berechnung der aktuellen Stellung und eine genaue Steuerung der Endoskopspitze bzw. der Spitze des endoskopischen Instruments.

Eine flexible Gewindestange, die insbesondere zur Verwendung in einem oben beschriebenen Gewindetrieb geeignet ist, kann beispielsweise durch Aufschweißen eines schraubenförmig gewendelten Drahts auf einen flexiblen Draht hergestellt werden. Der Querschnitt des gewendelten Drahts entspricht dabei dem gewünschten Gewindeprofil, während die Steigung der Wendel der Gewindesteigung entspricht. Dabei kann beispielsweise eine dreiecksförmige Querschnittsfläche besonders vorteilhaft sein, wodurch einerseits eine sichere Befestigung auf dem flexiblen Draht ermöglicht wird und andererseits ein Gewinde mit vorteilhaften Eigenschaften erzeugt wird. Der gewendelte Draht kann bereits vor dem Aufschweißen schraubenförmig gewendelt sein oder erst durch das Aufschweißen gewendelt werden. Anstelle des Aufschweißens sind auch andere Verbindungsarten, etwa Löten oder Kleben, möglich. Dadurch kann auf einfache und kostengünstige Weise eine flexible Gewindestange mit einem geringen Durchmesser und einer gewünschten Steigung und einem gewünschten Gewindeprofil hergestellt werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1a und 1b ein erfindungsgemäßes flexibles Endoskop gemäß einem Ausführungsbeispiel in einer Gesamtansicht;
Fig. 2 einen schematischen Längsschnitt durch einen Endbereich eines Schafts gemäß einem ersten Ausführungsbeispiel der Erfindung, wobei nur eine Hälfte des Längsschnitts dargestellt ist;
Fig. 3 einen schematischen Längsschnitt durch einen Endbereich eines Schafts gemäß einem zweiten Ausführungsbeispiel der Erfindung, wobei nur eine Hälfte des Längsschnitts dargestellt ist;
Fig. 4 eine erst teilweise mit einem Gewinde versehene flexible Gewindestange gemäß einem Ausführungsbeispiel;
Fig. 5 eine schematische Darstellung eines Kopplungsmechanismus zwischen einem Endoskopschaft und einer Handhabe gemäß einem Ausführungsbeispiel der Erfindung.

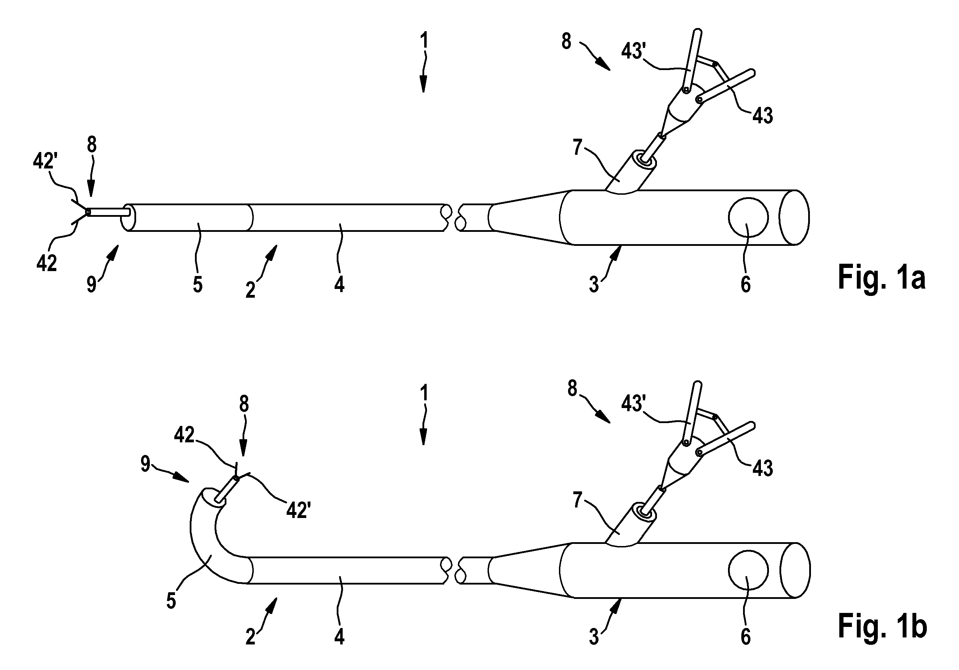

Wie in Fig. 1a und 1b dargestellt ist, umfasst ein erfindungsgemäßes flexibles Endoskop 1 gemäß einem Ausführungsbeispiel der Erfindung einen flexiblen Endoskopschaft 2 und einen Handgriff 3. Der Endoskopschaft 2 umfasst einen ersten Schaftabschnitt 4 und einen zweiten Schaftabschnitt 5. Der zweite Schaftabschnitt 5 ist der distale Endabschnitt des Endoskopschafts 2, während der erste Schaftabschnitt 4 der proximale Teil des Endoskopschafts 2 ist, der im gezeigten Beispiel die gesamte übrige Länge des Endoskopschafts 2 einnimmt und deutlich länger als der zweite Schaftabschnitt 5 ist. Am Handgriff 3 ist ein symbolisch dargestelltes Bedienelement 6 zum Steuern einer Abwinkelung des zweiten Schaftabschnitts 5 angeordnet. Das Bedienelement 6 kann etwa als Handrad, Hebel oder Kurbel ausgebildet sein, kann aber auch beispielsweise ein Bedienelement zum Ansteuern eines Motors sein. Ferner weist der Handgriff 3 ein Führungsrohr 7 auf, in das ein beispielhaft dargestelltes, nicht erfindungsgemäß steuerbares endoskopisches Arbeitsinstrument 8 eingeführt und durch einen sich innerhalb des Endoskopschafts 2 erstreckenden Arbeitskanal bis zum distalen Ende 9 des Endoskopschafts 2 und darüber hinaus geführt werden kann. Das Arbeitsinstrument 8 weist an seinem distalen Ende ein beispielsweise zum Greifen oder Schneiden ausgebildetes Werkzeug mit zwei gegeneinander wirkenden Maulteilen 42, 42' auf, die über proximalseitig angeordnete Griffe 43, 43' betätigt werden können.

In Fig. 1a ist der Endoskopschaft 2 in einem gestreckten Zustand dargestellt, während Fig. 1b das Endoskop 1 der Fig. 1a in einem abgewinkelten Zustand zeigt. Das Instrument 8 und der erste Schaftabschnitt 4 sind flexibel ausgebildet, jedoch nicht aktiv steuerbar. Demgegenüber ist der zweite Schaftabschnitt 5 des Endoskopschafts 2 durch Betätigung des Bedienelements 6 aktiv krümmbar und damit steuerbar. Der zweite Schaftabschnitt 5 weist in seinem Inneren eine Stützstruktur aus gelenkig miteinander verbundenen Schaftsegmenten auf, die zur Abwinkelung mit mindestens zwei einander gegenüberliegenden, innerhalb des zweiten Schaftabschnitts 5 geführten Zugmitteln zusammenwirken. Jedes der Zugmittel ist durch jeweils eine Torsionswelle, die durch den ersten Schaftabschnitt 4 verläuft, mit dem Handgriff 3 und dem Bedienelement 6 verbunden. Der Endoskopschaft 2 kann über eine Kupplung von dem Handgriff 3 trennbar und mit diesem wieder verbindbar sein (nicht dargestellt).

Der Endoskopschaft 2 kann abschnittsweise durch ein umgebendes Metallgeflecht verstärkt sein. Der erste Schaftabschnitt 4 und der zweite Schaftabschnitt 5 können von einer flexiblen Hülle, die beispielsweise ein Schlauch aus einem Kunststoffmaterial sein kann, umgeben sein. Der distale Endbereich des zweiten Schaftabschnitts 5 kann starr ausgebildet sein und optische und elektronische Bauelemente aufnehmen, etwa ein Endoskopobjektiv sowie ggf. einen elektronischen Bildaufnehmer (nicht dargestellt).

Ein erfindungsgemäßes flexibles endoskopisches Instrument ist gemäß einem Ausführungsbeispiel aufgebaut wie das in Fig. 1a und 1b dargestellte flexible Endoskop 1, weist jedoch keine Optik auf. Das flexible endoskopische Instrument kann das endoskopische Arbeitsinstrument 8 umfassen oder auch als endoskopisches Führungsinstrument zur Führung und Steuerung eines nicht steuerbaren endoskopischen Arbeitsinstruments 8 ausgebildet sein.

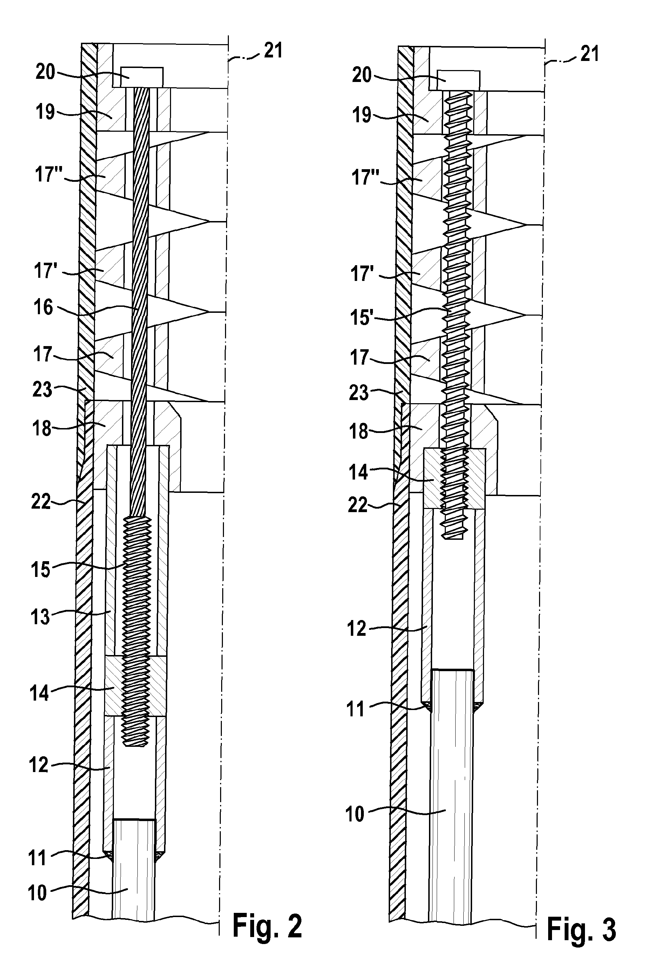

In Fig. 2 ist eine Ausführungsform der Erfindung mit einem als Seilzug ausgebildeten Zugmittel des zweiten Schaftabschnitts 5 des Endoskopschafts 2 (s. Fig. 1), das über einen Gewindetrieb mit der im ersten Schaftabschnitt 4 angeordneten Torsionswelle 10 verbunden ist, im Längsschnitt dargestellt, wobei nur die auf einer Seite der Längsachse 21 gelegene Hälfte des Längsschnitts gezeigt ist. Die Torsionswelle 10 kann an ihrem proximalen Ende beispielsweise motorisch, insbesondere durch einen in einem nicht dargestellten Handgriff angeordneten Elektromotor, rotatorisch angetrieben werden. Die Torsionswelle 10 ist langerstreckt, dünn und möglichst rotationssteif ausgebildet. Die Torsionswelle 10 kann etwa aus Nitinol hergestellt sein. Die Torsionswelle 10 kann beispielsweise als dünnes, flexibles Rohr ausgeführt sein. Am distalen Ende der Torsionswelle 10 ist diese fest mit einer Hülse 12 verbunden, etwa mit Hilfe eines Klebstoffs 11, der einen Ringspalt und eine Hohlkehle, die von der Hülse 12 mit der Torsionswelle 10 gebildet werden, ausfüllt. Die Hülse 12 ist drehbar innerhalb des ersten Schaftabschnitts 4 gelagert und durch eine Führung 13 gegen eine Längsverschiebung, insbesondere in distaler Richtung, abgestützt. Die Hülse 12 ist an ihrem distalen Endbereich fest mit einer Mutter 14 verbunden, die auf einer Gewindestange 15 läuft, wobei ein Innengewinde der Mutter 14 in ein Außengewinde der Gewindestange 15 eingreift. Die Gewindestange 15 ist längsverschiebbar gelagert und kann durch die Führung 13 und/oder über den unten beschriebenen Zugdraht 16, der weitgehend rotationssteif ausgebildet sein kann, und die im Endsegment 19 drehfest gelagerte Halterung 20 gegen Verdrehung gesichert sein. Die Gewindestange 15 ist relativ kurz, etwa mit einer Länge von 15 mm, und starr ausgebildet. Der Gewindetrieb, der durch die Hülse 12 mit der Mutter 14, die Führung 13 und die Gewindestange 15 gebildet wird, ist in einem distalen Endbereich des ersten Schaftabschnitts 4 angeordnet. Dieser kann ohne wesentliche Einschränkung der Funktionalität des Endoskops starr oder mit einer geringen Flexibilität ausgebildet sein, wodurch eine einfache Ausbildung des Gewindetriebs ermöglicht wird.

Die Gewindestange 15 ist zugfest und vorzugsweise drehfest mit einem Zugdraht 16 verbunden, der in einem Randbereich des zweiten Schaftabschnitts 5 geführt ist. Der zweite Schaftabschnitt 5 weist eine Stützstruktur auf, von der in Fig. 2 symbolisch drei Segmente 17, 17', 17" sowie ein proximales Endsegment 18 und ein distales Endsegment 19 dargestellt sind. Die Führung 13 des Gewindetriebs ist im proximalen Endsegment 18 fixiert bzw. gelagert. Am distalen Endsegment 19 ist der Zugdraht 16 durch eine Halterung 20 zugfest und vorzugsweise auch drehfest gehalten. Das distale Endsegment 19 kann weitere Bauteile aufweisen, beispielsweise ein Endoskopobjektiv und/oder Ausgänge von innerhalb des Endoskopschafts verlaufenden Kanälen. Der Zugdraht 16 ist im Randbereich der Segmente 18, 17, 17', 17", 19 durch entsprechende Öffnungen bzw. Bohrungen längsverschiebbar geführt. Wie in Fig. 2 zu erkennen ist, ist die Halterung 20 des Zugdrahts 16 gegenüber einer Längsachse 21 des zweiten Schaftabschnitts 5 radial versetzt und damit auch gegenüber den Schwenkachsen, mit denen die Segmente 18, 17, 17', 17", 19 gegeneinander schwenkbar gelagert sind und von denen beispielsweise abwechselnd eine im Wesentlichen senkrecht zur Längsachse 21 steht und diese schneidet und die Schwenkachsen der jeweils benachbarten Segmente senkrecht dazu stehen, so dass die unterschiedlich gerichteten Schwenkachsen eine Abwinkelung in alle Richtungen ermöglichen.

Wird nun die Torsionswelle 10 durch den in Fig. 2 nicht dargestellten Motor in Rotation versetzt, so wird die Mutter 14 von der Hülse 12 mitgenommen und gegenüber der drehfest im Endoskopschaft geführten distalen Gewindestange 15 gedreht. Bei einer Rotation in einer ersten Rotationsrichtung wird die Gewindestange 15 in proximaler Richtung verschoben; das proximale Ende der Gewindestange 15 wird dann proximalseitig der Mutter 14 in der Hülse 12 aufgenommen. Der Zugdraht 16 wird dabei gegenüber dem proximalen Endsegment 18 in proximaler Richtung verschoben. Aufgrund der außeraxialen Anordnung der Halterung 20 wird dadurch eine Kippung des distalen Endsegments 19 und, je nach Ausgestaltung der gelenkigen Verbindung zwischen den Segmenten 18, 17, 17', 17", 19, auch eine entsprechende Kippung der Segmente 17, 17', 17" bewirkt. Hierdurch entsteht eine Krümmung des zweiten Schaftabschnitts 5 und dadurch eine Abwinkelung des zweiten Schaftabschnitts 5 bzw. der durch das distale Endsegment 19 gebildeten Endoskopspitze.

Eine umgekehrte Rotation der Torsionswelle 10 führt zu einer Verschiebung der Gewindestange 15 in distaler Richtung und somit zu einer Entspannung des Zugdrahts 16. Ist auf der Seite des Endoskopschafts, die der in Fig. 2 dargestellten Seite radial gegenüber liegt, ein weiterer, ähnlicher Mechanismus wie in Fig. 2 dargestellt angeordnet, so kann durch eine Rotation der auf der gegenüberliegenden Seite angeordneten Torsionswelle eine Abwinkelung des zweiten Schaftabschnitts 5 in der entgegengesetzten Richtung bewirkt werden, der der Zugdraht 16 folgt. Sofern die Halterung 20, der Zugdraht 16 und die Führung des Zugdrahts 16 in den Schaftsegmenten 18, 17, 17', 17", 19 entsprechend ausgebildet sind, kann auch eine Schubkraft zur Abwinkelung des zweiten Schaftabschnitts 5 in entgegengesetzter Richtung ausgeübt werden. Um eine Abwinkelung des zweiten Schaftabschnitts 5 in alle Richtungen zu ermöglichen, können vier paarweise einander entgegenwirkende Gewindetriebe mit jeweils einer Torsionswelle 10 vorgesehen sein, die bezüglich der Längsachse 21 jeweils um 90° zueinander versetzt sind. In Fig. 2 sind auch eine Außenhülle 22, die den ersten Schaftabschnitt 4 umschließt, und die mit dieser verbundene Außenhülle 23, die den zweiten Schaftabschnitt 5 umschließt, angedeutet.

In Fig. 3 ist ebenfalls als halbseitiger Längsschnitt eine Ausführungsform der Erfindung dargestellt, bei der kein Zugdraht zur Bewirkung der Abwinkelung des zweiten Schaftabschnitts 5 benötigt wird, sondern anstelle des Zugdrahts eine flexible Gewindestange 15' vorgesehen ist, die im zweiten Schaftabschnitt 5 geführt ist. Wie zu Fig. 2 beschrieben, verläuft innerhalb des ersten Schaftabschnitts 4 eine Torsionswelle 10, die an ihrem proximalen Ende beispielsweise elektromotorisch antreibbar ist. Mit der Torsionswelle 10 ist mittels des Klebstoffs 11 eine drehbare Hülse 12 verbunden, die an ihrem distalen Endbereich fest mit einer Mutter 14 verbunden ist. Die Mutter 14 ist in einem proximalen Endsegment 18 des zweiten Schaftabschnitts 5 drehbar, jedoch zumindest in distaler Richtung nicht längsverschiebbar gelagert. Das Innengewinde der Mutter 14 greift in das Außengewinde der flexiblen Gewindestange 15' ein. Die Gewindestange 15' ist längsverschiebbar in den Öffnungen bzw. Bohrungen in den Randbereichen der Schaftsegmente 17, 17', 17" sowie des proximalen Endsegments 18 geführt. Die Gewindestange 15' kann in einer flexiblen Hülle geführt sein. Mit ihrem distalen Ende ist die Gewindestange 15' in einer Halterung 20 dreh- und zugfest am distalen Endsegment 19 gehalten.

Durch eine Drehung der Mutter 14 aufgrund einer Rotation der Torsionswelle 10 wird die Gewindestange 15' je nach Drehrichtung mit ihrem proximalen Endbereich in proximaler oder distaler Richtung verschoben. Hierdurch wird eine Zug- bzw. eine Druckkraft auf das distale Endsegment ausgeübt, wobei durch die außeraxiale Führung der Gewindestange 15' eine entsprechende Abwinkelung des zweiten Schaftabschnitts 5 bewirkt wird. Ein auf der radial gegenüber liegenden Seite angeordneter, ähnlich aufgebauter Mechanismus kann der Gewindestange 15' entgegenwirken, so dass auch allein durch Zugkräfte eine Abwinkelung in jeder Richtung bewirkt werden kann. Die in Fig. 3 dargestellte Ausführungsform, bei der das Zugmittel selbst als Gewindestange 15' ausgebildet ist bzw. die Gewindestange 15' selbst als Zugdraht wirkt, arbeitet somit in ähnlicher Weise wie die in Fig. 2 dargestellte, kommt jedoch ohne einen separaten Seilzug aus und ist daher besonders einfach aufgebaut.

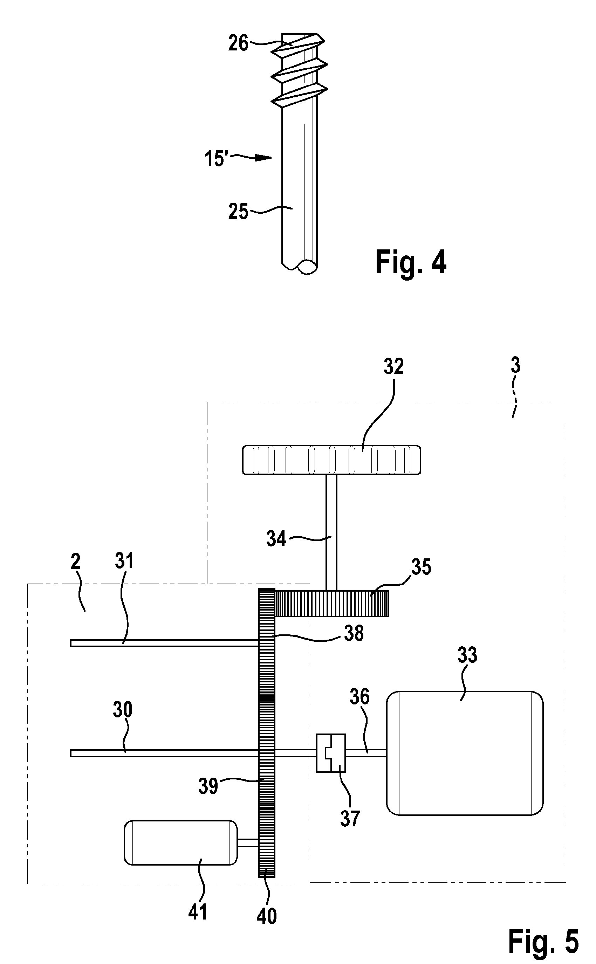

In Fig. 4 ist eine flexible Gewindestange 15', die insbesondere zur Verwendung in einer Ausführungsform gemäß Fig. 3 geeignet ist, gemäß einem beispielhaft beschriebenen Herstellungsverfahren in einem erst teilweise mit einem Gewinde versehenen Zustand gezeigt. Eine solche flexible Gewindestange 15' kann durch Aufschweißen eines Drahts 26 auf einen flexiblen Draht 25 hergestellt werden. Der Querschnitt des aufgeschweißten Drahts 26 entspricht dabei dem gewünschten Gewindeprofil, etwa einem dreiecksförmigen Profil. Beim Aufschweißen wird der Draht 26 mit einer flachen Seite auf dem Draht 25 in Form einer Schraubenlinie fixiert, wobei die Steigung der Schraubenlinie die Gewindesteigung der Gewindestange 15' ergibt. Steigung und Profil des Innengewindes der Mutter 14 sind an das auf diese Weise hergestellte Außengewinde der Gewindestange 15' angepasst.

Wie in Fig. 5 symbolisch dargestellt, kann ein Endoskopschaft 2 eine erste und eine zweite Torsionswelle 30, 31 aufweisen, die sowohl über ein Handrad 32 als auch über einen Motor 33 antreibbar sind. Das Handrad 32 ist über eine Welle 34 mit einem Zahnrad 35 verbunden; dabei kann eine in Fig. 4 nicht dargestellte Untersetzung vorgesehen sein, wodurch beispielsweise eine Umdrehung des Handrads 5 bis 10 Umdrehungen der Torsionswellen bewirkt. Der Motor 33 treibt eine Motorwelle 36 an, die eine Kupplung 37 aufweist. Die Kupplung kann beispielsweise als Schleifkupplung, Magnetkupplung, Zapfwelle oder in anderer, an sich bekannter Weise ausgebildet sein. Handrad 32 und Motor 33 sowie die Wellen 34 und 36, das Zahnrad 35 und die Kupplung 37 sind einem Handgriff 3 zugeordnet, der an den Endoskopschaft 2 angesetzt werden kann.

Beim Ansetzen an den Endoskopschaft 2 kämmt das Zahnrad 35 mit einem Zahnrad 38, das mit der Torsionswelle 31 drehfest verbunden ist und diese antreibt. Über die Kupplung 37 wird die Torsionswelle 30 direkt angetrieben. Das Zahnrad 38 kämmt mit dem Zahnrad 39, das drehfest auf der Torsionswelle 30 sitzt und das wiederum das Zahnrad 40 antreibt, das mit einem Drehgeber 41 verbunden ist. Durch die Verbindung der Zahnräder 38, 39 ist die relative Rotation der Torsionswellen 30, 31 definiert. Dabei haben die Gewindestangen der über die Torsionswellen 30, 31 angetriebenen Gewindetriebe gleichsinnige Steigungen, so dass bei einer Verkürzung auf einer Seite des zweiten Schaftabschnitts 5 eine entsprechende Verlängerung auf der anderen Seite des zweiten Schaftabschnitts 5 bewirkt wird. Der Drehgeber 41 registriert die Drehung des Zahnrads 40, woraus sich eine Drehposition der Torsionswellen 30, 31 ermitteln lässt. In einer nicht dargestellten Speichervorrichtung wird diese Drehposition gespeichert und beim Ankoppeln des Schafts 2 an den Handgriff 3 an eine ebenfalls nicht dargestellte Steuerungseinrichtung des Motors 33 übermittelt. Hierdurch kann jederzeit eine der tatsächlichen Stellung der Antriebe entsprechende Ansteuerung des Motors 33 erreicht werden.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Endoskop
- 2: Endoskop schaft
- 3: Handgriff
- 4: Erster Schaftabschnitt
- 5: Zweiter Schaftabschnitt
- 6: Bedienelement
- 7: Führungsrohr
- 8: Endoskopisches Arbeitsinstrument
- 9: Distales Ende
- 10: Torsionswelle
- 11: Klebstoff
- 12: Hülse
- 13: Führung
- 14: Mutter
- 15, 15': Gewindestange
- 16: Zugdraht
- 17, 17', 17": Schaftsegment
- 18: Proximales Endsegment
- 19: Distales Endsegment
- 20: Halterung
- 21: Längsachse
- 22: Außenhülle
- 23: Außenhülle
- 25: Draht
- 26: Draht
- 30: Torsionswelle
- 31: Torsionswelle
- 32: Handrad
- 33: Motor
- 34: Welle
- 35: Zahnrad
- 36: Motorwelle
- 37: Kupplung
- 38: Zahnrad
- 39: Zahnrad
- 40: Zahnrad
- 41: Drehgeber
- 42, 42': Maulteil
- 43, 43': Griff

## Patentansprüche

1. Schaft für ein flexibles Endoskop oder für ein flexibles endoskopisches Instrument, der einen ersten Schaftabschnitt (4) und einen distalseitig des ersten angeordneten zweiten Schaftabschnitt (5) umfasst, wobei der zweite Schaftabschnitt (5) relativ zu einem distalen Endbereich des ersten Schaftabschnitts (4) durch eine Längsverstellung mindestens eines innerhalb des zweiten Schaftabschnitts (5) längsverschiebbar geführten und mit einem distalen Endbereich des zweiten Schaftabschnitts (5) verbundenen Zugmittels abwinkelbar ist, wobei zur Längsverstellung des mindestens einen Zugmittels mindestens ein Gewindetrieb vorgesehen ist, der durch mindestens eine innerhalb des ersten Schaftabschnitts (4) verlaufende Torsionswelle (10, 30, 31) antreibbar ist, **dadurch gekennzeichnet, dass** die Torsionswelle lang erstreckt, durchgehend vom proximalen zum distalen Endbereich des ersten Schaftabschnitts und zumindest abschnittsweise flexibel ausgebildet ist.

2. Schaft nach Anspruch 1, **dadurch gekennzeichnet, dass** das mindestens eine Zugmittel als Seilzug (16) ausgebildet und mit einem distalen Ende der Torsionswelle (10, 30, 31) über den Gewindetrieb verbunden ist.

3. Schaft nach Anspruch 2, **dadurch gekennzeichnet, dass** der Gewindetrieb eine Gewindestange (15) umfasst, die mit einer mit dem distalen Ende der Torsionswelle (10, 30, 31) drehfest verbundenen Mutter (14) zur Längsverstellung des Seilzugs (16) zusammenwirkt.

4. Schaft nach Anspruch 3, **dadurch gekennzeichnet, dass** die Gewindestange (15) mit einem proximalen Ende des Seilzugs (16) verbunden ist und im distalen Endbereich des ersten Schaftabschnitts (4) längsverschiebbar und drehfest gelagert ist.

5. Schaft nach Anspruch 1, **dadurch gekennzeichnet, dass** das mindestens eine Zugmittel als zumindest abschnittsweise flexible Gewindestange (15') des mindestens einen Gewindetriebs ausgebildet ist, die mit einer mit dem distalen Ende der Torsionswelle (10, 30, 31) drehfest verbundenen Mutter (14) zur Längsverstellung der flexiblen Gewindestange (15') zusammenwirkt.

6. Schaft nach einem der Ansprüche 1 bis 5, **dadurch gekennzeichnet, dass** der zweite Schaftabschnitt (5) eine Mehrzahl von Zugmitteln aufweist, die einander paarweise gegenüber liegen.

7. Schaft nach Anspruch 6, **dadurch gekennzeichnet, dass** der erste Schaftabschnitt (4) eine Mehrzahl von Torsionswellen (10, 30, 31) aufweist, die mit der Mehrzahl von Zugmitteln zur Steuerung einer Abwinkelung in unterschiedliche Richtungen zusammenwirken.

8. Schaft nach Anspruch 7, **dadurch gekennzeichnet, dass** die Mehrzahl von Torsionswellen (10, 30, 31) über mindestens ein Zahnradgetriebe derart miteinander verbunden sind, dass einander gegenüberliegende Zugmittel gegensinnig längsverstellbar sind.

9. Flexibles Endoskop mit einem Schaft nach einem der vorhergehenden Ansprüche.

10. Flexibles endoskopisches Instrument mit einem Schaft nach einem der vorhergehenden Ansprüche.

11. Flexibles Endoskop nach Anspruch 9 oder flexibles endoskopisches Instrument nach Anspruch 10, **dadurch gekennzeichnet, dass** die Torsionswelle (10, 30, 31) über mindestens ein proximalseitig des ersten Schaftabschnitts angeordnetes Bedienelement (6) und/oder einen proximalseitig des ersten Schaftabschnitts angeordneten Motor (33) betätigbar ist.

12. Flexibles Endoskop nach Anspruch 9 oder flexibles endoskopisches Instrument nach Anspruch 10, **dadurch gekennzeichnet, dass** das flexible Endoskop bzw. das flexible endoskopische Instrument eine proximalseitig des ersten Schaftabschnitts (4) angeordnete Handhabe umfasst, und dass der Schaft mit der Handhabe lösbar verbindbar ist.

13. Flexibles Endoskop oder flexibles endoskopisches Instrument nach Anspruch 12, **dadurch gekennzeichnet, dass** der Handhabe ein motorischer Antrieb der mindestens einen Torsionswelle (10, 30, 31) zugeordnet ist, dass eine rotatorische Position der mindestens einen Torsionswelle (10, 30, 31) beim Trennen des Schafts von der Handhabe speicherbar ist und dass eine Torsionswellenkupplung der Handhabe zum Ankoppeln des Schafts entsprechend ansteuerbar ist.

14. Flexibles Endoskop oder flexibles endoskopisches Instrument nach Anspruch 13, **dadurch gekennzeichnet, dass** die rotatorische Position der mindestens einen Torsionswelle (10, 30, 31) in einem dem Schaft zugeordneten Speicher speicherbar ist.

15. Flexibles Endoskop oder flexibles endoskopisches Instrument nach Anspruch 13 oder 14, **dadurch gekennzeichnet, dass** zur Ermittlung der rotatorischen Position ein Potentiometer oder ein Drehgeber (41) vorgesehen ist.

## Claims

1. Shank for a flexible endoscope or for a flexible endoscopic instrument, comprising a first shank portion (4) and, arranged in the distal direction from the first shank portion, a second shank portion (5), wherein the second shank portion (5) can be angled relative to a distal end region of the first shank portion (4) by a longitudinal adjustment of at least one tensioning means which is guided in a longitudinally displaceable manner inside the second shank portion (5) and connected to a distal end region of the second shank portion (5), wherein, for the longitudinal adjustment of the at least one tensioning means, provision is made for at least one threaded drive, which can be driven by at least one torsion shaft (10, 30, 31) extending inside the first shank portion (4), **characterized in that** the torsion shaft is elongate and continuous from the proximal to the distal end region of the first shank portion and has a flexible design, at least in portions thereof.

2. Shank according to Claim 1, **characterized in that** the at least one tensioning means is embodied as a cable pull (16) and connected to a distal end of the torsion shaft (10, 30, 31) by the threaded drive.

3. Shank according to Claim 2, **characterized in that** the threaded drive comprises a threaded rod (15), which, for longitudinal adjustment of the cable pull (16), cooperates with a nut (14) connected to the distal end of the torsion shaft (10, 30, 31) for rotation therewith.

4. Shank according to Claim 3, **characterized in that** the threaded rod (15) is connected to a proximal end of the cable pull (16) and mounted in a longitudinally displaceable and rotationally secured manner in the distal end region of the first shank portion (4).

5. Shank according to Claim 1, **characterized in that** the at least one tensioning means is embodied as a threaded rod (15'), which is flexible at least in portions, of the at least one threaded drive, the threaded rod cooperating with a nut (14) connected to the distal end of the torsion shaft (10, 30, 31) for rotation therewith for longitudinal adjustment of the flexible threaded rod (15').

6. Shank according to one of Claims 1 to 5, **characterized in that** the second shank portion (5) has a plurality of tensioning means, which lie opposite to one another in pairs.

7. Shank according to Claim 6, **characterized in that** the first shank portion (4) has a plurality of torsion shafts (10, 30, 31), which cooperate with the plurality of tensioning means for controlling angling in different directions.

8. Shank according to Claim 7, **characterized in that** the plurality of torsion shafts (10, 30, 31) are connected to one another by at least one toothed gear such that tensioning means lying opposite to one another can be longitudinally adjusted in opposite senses.

9. Flexible endoscope with a shank according to one of the preceding claims.

10. Flexible endoscopic instrument with a shank according to one of the preceding claims.

11. Flexible endoscope according to Claim 9 or flexible endoscopic instrument according to Claim 10, **characterized in that** the torsion shaft (10, 30, 31) can be actuated by at least one operating element (6) arranged on the proximal side of the first shank portion and/or by a motor (33) arranged on the proximal side of the first shank portion.

12. Flexible endoscope according to Claim 9 or flexible endoscopic instrument according to Claim 10, **characterized in that** the flexible endoscope or the flexible endoscopic instrument comprises a handle arranged on the proximal side of the first shank portion (4) and **in that** the shank is detachably connectable to the handle.

13. Flexible endoscope or flexible endoscopic instrument according to Claim 12, **characterized in that** the handle is associated with a motor-driven drive of the at least one torsion shaft (10, 30, 31), **in that** a rotary position of the at least one torsion shaft (10, 30, 31) can be stored when separating the shank from the handle and **in that** a torsion shaft coupling of the handle can be actuated accordingly for coupling on the shank.

14. Flexible endoscope or flexible endoscopic instrument according to Claim 13, **characterized in that** the rotary position of the at least one torsion shaft (10, 30, 31) can be stored in a storage medium associated with the shank.

15. Flexible endoscope or flexible endoscopic instrument according to Claim 13 or 14, **characterized in that** a potentiometer or a rotary encoder (41) is provided for establishing the rotary position.

## Revendications

1. Tige pour un endoscope flexible ou pour un instrument endoscopique flexible, qui comprend une première partie de tige (4) et une deuxième partie de tige (5) disposée du côté distal de la première, dans laquelle la deuxième partie de tige (5) peut être pliée par rapport à une région d'extrémité distale de la première partie de tige (4) par un déplacement longitudinal d'au moins un moyen de traction guidé de manière déplaçable longitudinalement à l'intérieur de la deuxième partie de tige (5) et connecté à une région d'extrémité distale de la deuxième portion de tige (5), dans laquelle il est prévu pour le déplacement longitudinal dudit au moins un moyen de traction au moins une vis d'entraînement, qui peut être entraînée par au moins un arbre de torsion (10, 30, 31) s'étendant à l'intérieur de la première portion de tige (4), **caractérisée en ce que** l'arbre de torsion est réalisé sous forme allongée de manière continue depuis la région d'extrémité proximale jusqu'à la région. d'extrémité distale de la première portion de tige et de manière au moins en partie flexible.

2. Tige selon la revendication 1, **caractérisée en ce que** ledit au moins un moyen de traction est constitué par un câble Bowden (16) et est attaché à une extrémité distale de l'arbre de torsion (10, 30, 31) au moyen de la vis d'entraînement.

3. Tige selon la revendication 2, **caractérisée en ce que** la vis d'entraînement comprend une tige filetée (15) qui coopère avec un écrou (14) connecté de manière solidaire en rotation à l'extrémité distale de l'arbre de torsion (10, 30, 31), pour le réglage en longueur du câble Bowden (16).

4. Tige selon la revendication 3, **caractérisée en ce que** la tige filetée (15) est connectée à une extrémité proximale du câble Bowden (16) et est déplaçable longitudinalement et supportée de manière solidaire en rotation dans la région d'extrémité distale de la première portion de tige (4).

5. Tige selon la revendication 1, **caractérisée en ce que** l'au moins un moyen de traction est réalisé sous forme de tige filetée (15') au moins en partie flexible de l'au moins une vis d'entraînement, qui coopère avec un écrou (14) connecté de manière solidaire en rotation à l'extrémité distale de l'arbre de torsion (10, 30, 31) pour le réglage en longueur de la tige filetée flexible (15').

6. Tige selon l'une quelconque des revendications 1 à 5, **caractérisé en ce que** la deuxième partie de tige (5) présente une multiplicité de moyens de traction, qui sont opposés l'un à l'autre par paires.

7. Tige selon la revendication 6, **caractérisée en ce que** la première partie de tige (4) présente une multiplicité d'arbres de torsion (10, 30, 31), qui coopèrent avec la multiplicité de moyens de traction pour la commande d'un pliage dans différentes directions.

8. Tige selon la revendication 7, **caractérisée en ce que** la multiplicité d'arbres de torsion (10, 30, 31) sont reliés les uns aux autres par au moins une transmission à roues dentées, de telle manière que des moyens de traction opposés l'un à l'autre soient déplaçables en longueur en sens contraire.

9. Endoscope flexible avec une tige selon l'une quelconque des revendications précédentes.

10. Instrument endoscopique flexible avec une tige selon l'une quelconque des revendications précédentes.

11. Endoscope flexible selon la revendication 9 ou instrument endoscopique flexible selon la revendication 10, **caractérisé en ce que** l'arbre de torsion (10, 30, 31) peut être actionné par au moins un élément de commande (6) disposé du côté proximal de la première partie de tige et/ou par un moteur (33) disposé du côté proximal de la première partie de tige.

12. Endoscope flexible selon la revendication 9 ou instrument endoscopique flexible selon la revendication 10, **caractérisé en ce que** l'endoscope flexible ou l'instrument endoscopique flexible comprend un manche disposé du côté proximal de la première partie de tige (4), et **en ce que** la tige est assemblée au manche de façon détachable.

13. Endoscope flexible ou instrument endoscopique flexible selon la revendication 12, **caractérisé en ce qu'**un entraînement motorisé dudit au moins un arbre de torsion (10, 30, 31) est associé au manche, **en ce qu'**une position angulaire dudit au moins un arbre de torsion (10, 30, 31) peut être mémorisée par le manche lors de la séparation de la tige et **en ce qu'**un couplage d'arbre de rotation du manche peut être commandé de façon correspondante pour le couplage de la tige.

14. Endoscope flexible ou instrument endoscopique flexible selon la revendication 13, **caractérisé en ce que** la position angulaire dudit au moins un arbre de torsion (10, 30, 31) peut être mémorisée dans une mémoire associée à la tige.

15. Endoscope flexible ou instrument endoscopique flexible selon la revendication 13 ou 14, **caractérisé en ce qu'**il est prévu un potentiomètre ou un capteur de rotation (41) pour la détermination de la position angulaire.
